# EUROPEAN PATENT SPECIFICATION

(11) **EP 1 809 196 B1**
(45) Date of publication and mention of the grant of the patent: **09.06.2010**
(21) Application number: 05810478.7
(22) Date of filing: 12.10.2005
(51) Int. Cl.: A61B 18/18, A61F 9/007

(54) **CAPSULARHEXIS DEVICE**
KAPSULARHEXIS-VORRICHTUNG
DISPOSITIF DE CAPSULO-RHEXIS

(30) Priority: 09.11.2004 US 984383
(43) Date of publication of application: 25.07.2007
(73) Proprietor: Alcon, Inc., 6331 Hünenberg (CH)
(72) Inventor: BOUKHNY, Mikhail, Laguna Niguel, CA 92677 (US)
(74) Representative: Hanna, Peter William Derek
(86) International application number: PCT/US2005/036670
(87) International publication number: WO 2006/052374

(56) References cited:
- WO-A-97/30669
- DE-A1- 19 740 530
- ES-A1- 2 103 635
- US-A- 4 481 948
- US-A- 5 766 171
- US-A- 6 066 138
- US-B1- 6 551 326

## Description

### Background of the Invention

This invention relates generally to the field of cataract surgery and more particularly to an apparatus for performing a capsularhexis.

The human eye in its simplest terms functions to provide vision by transmitting light through a clear outer portion called the cornea, and focusing the image by way of the lens onto the retina. The quality of the focused image depends on many factors including the size and shape of the eye, and the transparency of the cornea and lens.

When age or disease causes the lens to become less transparent, vision deteriorates because of the diminished light that can be transmitted to the retina. This deficiency in the lens of the eye is medically known as a cataract. An accepted treatment for this condition is surgical removal of the lens and replacement of the lens function by an artificial intraocular lens (IOL).

In the United States, the majority of cataractous lenses are removed by a surgical technique called phacoemulsification. A typical surgical handpiece suitable for phacoemulsification procedures consists of an ultrasonically driven handpiece, an attached cutting tip, and irrigating sleeve and an electronic control console. The handpiece assembly is attached to the control console by an electric cable and flexible tubings. Through the electric cable, the console varies the power level transmitted by the handpiece to the attached cutting tip and the flexible tubings supply irrigation fluid to and draw aspiration fluid from the eye through the handpiece assembly.

The operative part of the handpiece is a centrally located, hollow resonating bar or horn directly attached to a set of piezoelectric crystals. The crystals supply the required ultrasonic vibration needed to drive both the horn and the attached cutting tip during phacoemulsification and are controlled by the console. The crystal/horn assembly is suspended within the hollow body or shell of the handpiece by flexible mountings. The handpiece body terminates in a reduced diameter portion or nosecone at the body's distal end. The nosecone is externally threaded to accept the irrigation sleeve. Likewise, the horn bore is internally threaded at its distal end to receive the external threads of the cutting tip. The irrigation sleeve also has an internally threaded bore that is screwed onto the external threads of the nosecone. The cutting tip is adjusted so that the tip projects only a predetermined amount past the open end of the irrigating sleeve.

Prior to removing the cataractous lens, an opening, or rhexis, must be made in the anterior capsule. During phacoemulsification, there is a great deal of tension on the cut edges of the anterior capsularhexis while the lens nucleus is emulsified. Accordingly, for this method, a tagless, continuous cut or tear rhexis is a critical step for safe and effective phacoemulsification. If the capsule is opened with numerous small capsular tears, the small tags which remain can lead to radial capsular tears which may extend posteriorly through the posterior capsule. Such a radial tear constitutes a complication since it destabilizes the lens for further cataract removal and safe intraocular lens placement within the lens capsule later in the operation. More importantly, once the posterior capsule is punctured, the vitreous gains access to the anterior chamber of the eye. If the vitreous enters the front of the eye through a hole in the posterior capsule, the vitreous must be removed by an additional procedure with special instruments. The loss of vitreous also is associated with an increased rate of subsequent retinal detachment and/or infection within the eye. Importantly, these complications are potentially blinding. Prior art devices and methods used to produce a continuous curvilinear capsular opening require extraordinary skill and technique by the surgeon performing the operation. This is due to the extreme difficulty in controlling the path of the device.

For example, the most typical method begins with a capsular incision made with a cystotome. This incision is then coaxed to form a circular or oval shape by pushing the leading edge of the freshly tearing capsule with the cystotome in a non-cutting fashion. Alternatively, the initial capsular incision is torn into a circular shape by grasping the leading edge with fine caliber forceps and advancing the cut in a very uncontrolled manner. This is a very challenging maneuver and, even in the most experienced hands, the tearing motion can lead to an undesirable tear of the capsule toward the back of the lens. Moreover, even if a tagless edge is ultimately produced, the size and/or position of the capsular opening may present a problem. A small capsular opening can impair the safe removal of the lens nucleus and cortex and prevent proper intraocular lens insertion into the lens capsule. The additional stresses necessary to accomplish the operation with a small or misplaced capsular opening put the eye at risk for zonular and capsular breakage. Both of these types of breakage will likely increase the length and complexity of the operation and can result in vitreous loss.

A continuous, properly positioned and circular opening is highly desirable because it results in: (1) a significant reduction in radial tears and tags within the anterior capsule, (2) capsule integrity necessary for proper centering of a lens prosthesis; (3) safe and effective hydrodissection; and (4) safe use of capsular procedures on patients having poorly visualized capsules and/or small pupil openings. In addition, the capsularhexis must be properly sized relative to the diameter of the IOL being implanted in order to reduce the chances of a secondary cataract, also called posterior capsule opacification ("PCO") and for use with proposed accommodative IOL designs. Therefore, a need continues to exist for a device that can safely and effectively perform an anterior chamber capsularhexis.

DE 197 40 530 describes a capsularhexis device having a pair of handles that operate like tweezers to deform a ring into the shape of an oval. The ring comprises two arc-shaped support members made of plastic and two less rigid connecting parts which can be formed from wire electrodes, and which together act like a feather spring.

A capsularhexis device with a flexible manipulator connected to a flexible retractable ring at one end carrying an electrode is described in ES 2 103 635.

### Brief Summary of the Invention

The present invention provides a capsularhexis device in accordance with claims which follow. The capsularhexis device generally includes a flexible ring having a diameter suitable for an anterior chamber rhexis. Imbedded in the ring is a resistive wire as heating element or filament.

Accordingly, one objective of the present invention is to provide a capsularhexis device.

Another objective of the present invention is to provide a capsularhexis device capable of being inserted through a small incision.

These and other advantages and objectives of the present invention will become apparent from the detailed description and claims that follow.

### Brief Description of the Drawings

FIG. 1 is an enlarged, partial top plan view of the device of the present invention.
FIG. 2 is an enlarged, cross-sectional view of an embodiment which does not constitute a portion of the present invention taken at line 2-2 in FIG. 1.
FIG. 3 is a schematic illustration of the device of the present invention prior to insertion into an eye.
FIG. 4 is a schematic illustration of the device of the present invention inserted into an eye.
FIG. 5 is a schematic illustration of the device of the present invention being removed from an eye.
FIG. 6 is an enlarged cross-sectional view of a second embodiment of the present invention similar to FIG. 2.

### Detailed Description of the Invention

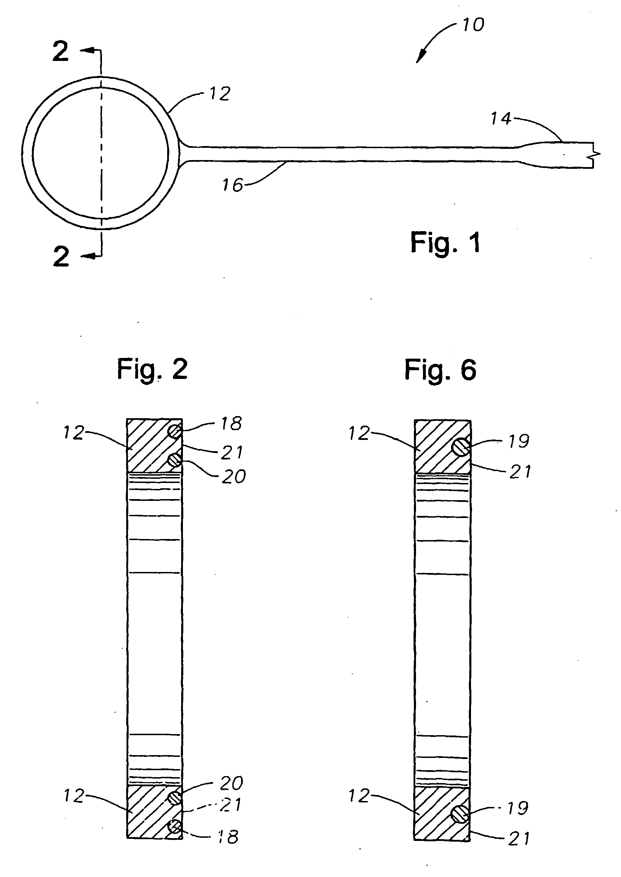

As seen in FIGS. 1 and 3-5, device 10 of the present invention generally include ring 12, handle 14 and shaft 16 connecting ring 12 with handle 14. Ring 12 may be made of any suitable flexible material such as an elastomer, acrylic or thermoplastic and may be any desired diameter, for example, between 3 mm and 6 mm. According to an embodiment which does not constitute a portion of the invention, there are embedded within ring 12 two adjacent electrodes 18 and 20 made from a flexible wire or other conductive material. As seen in FIG. 2, concentric electrodes 18 and 20 are located at posterior surface 21 of ring 12, and may even project out a small distance from surface 21. Ring 12 contains a resistive wire 19, as shown in FIG. 6, embedded in ring 12 in a manner similar to electrodes 18 and 20. Electrodes 18 and 20, and resistive wire 19 are connected to a source of electrical current (not shown) through shaft 16 and handle 14. Shaft 16 and handle 14 may be made from an inexpensive material, such as a thermoplastic.

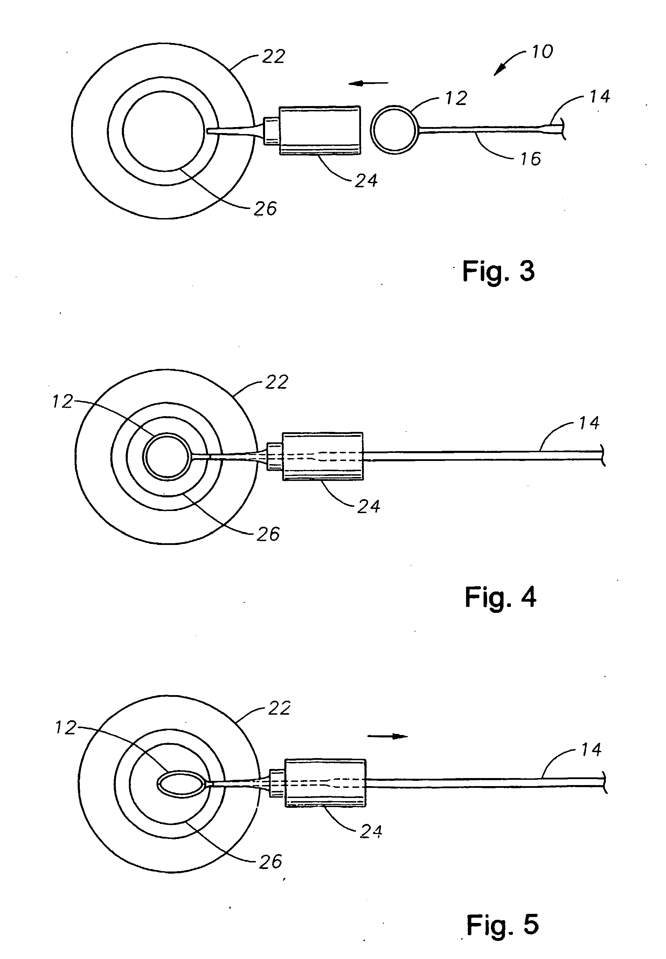

As best seen in FIGS. 3-5, prior to insertion into eye 22, ring 12 is expanded to its full diameter because of the flexible material used to form ring 12, such material also preferably have a shape memory. Ring 12 is easily collapsed (as seen in FIG. 5) so that it may be inserted into eye 22 through a small incision, for example, by use of intraocular lens insertion cartridge 24. Once within eye 22, ring 12 expands back to its full diameter. Following the formation of the capsularhexis, ring 12 may be withdrawn from eye 22 in a similar manner.

Once in the eye, as been seen in FIG. 4, ring 12 is pressed tightly against anterior capsule 26. Electrical current is supplied to electrodes 18 and 20, causing current to flow through capsule 26 between electrodes 18 and 20. This current flow causes localized heating of capsule 26 in the area between electrodes 18 and 20. Similar heating of capsule 26 is accomplished by applying electrical current to resistive wire 19. Such heating weakens capsule 26 and it may not be necessary to burn completely through capsule 26. Rather, this localized weaken area may define a boundary for the rhexis sufficient for the circular piece of capsule 26 to be removed using a conventional forceps, with little risk of radial tearing. Such heating also has a cauterizing effect on the rim of the rhexis, providing an additional resistance to radial tearing.

This description is given for purposes of illustration and explanation. It will be apparent to those skilled in the relevant art that changes and modifications may be made to the invention described above without departing from its scope.

## Claims

1. A capsularhexis device (10), adapted to be inserted into an eye incision comprising:
a) a handle (14) at one end of the device;
b) a flexible ring (12) having a posterior surface (21) at the other end of the device;
c) a resistive wire as a heating element (19) adapted to be connected to a source of electric current;
**characterized in that,**
the heating element (19) is embedded within the ring at the posterior surface (21) thereof, so as to project out a small distance from the surface (21).

2. The device of claim 1, wherein the flexible ring (12) is made of a relatively flexible material, including an elastomer, acrylic or thermoplastic.

3. The device of claim 2, wherein the flexible material used to form the ring (12) has a shape memory.

4. The device of claim 3, wherein the ring (12) is collapsible so that it may be inserted into an eye through an incision of less than 2mm.

5. A kit comprising the device of any of claims 1 to 4, and an intraocular lens insertion cartridge (24), wherein the device is adapted to be inserted through the intraocular lens insertion cartridge (24) by means of a shaft (16) connecting the ring (12) to the handle (14).

## Patentansprüche

1. Kapsularhexis-Vorrichtung (10), welche dazu ausgelegt ist, in einen Augeneinschnitt eingesetzt zu werden, welche enthält:
a) ein Griffstück (14) an einem Ende von der Vorrichtung;
b) einen flexiblen Ring (12), welcher eine hintere Oberfläche (21) hat, an dem anderen Ende von der Vorrichtung;
c) einen Widerstandsdraht als ein Erwärmungselement (19), welcher dazu ausgelegt ist, mit einer elektrischen Stromquelle verbunden zu werden;
**dadurch gekennzeichnet, dass**
das Erwärmungselement (19) innerhalb des Rings an der hinteren Oberfläche (21) davon eingebettet ist, um somit um eine geringe Distanz von der Oberfläche (21) hervorzuragen.

2. Vorrichtung nach Anspruch 1, bei welcher der flexible Ring (12) aus einem relativ flexiblen Material erstellt ist, welches ein Elastomer, ein Acryl oder einen Thermokunststoff enthält.

3. Vorrichtung nach Anspruch 2, bei welcher das flexible Material, welches dazu verwendet ist, um den Ring (12) auszubilden, ein Formgedächtnis hat.

4. Vorrichtung nach Anspruch 3, bei welcher der Ring (12) zusammenlegbar ist, sodass er durch einen Einschnitt von weniger als 2 mm in ein Auge eingesetzt werden kann.

5. Kombination, welche die Vorrichtung nach einem der Ansprüche 1 bis 4 und eine Intraokularlinse-Einsetzkassette (24) enthält, wobei die Vorrichtung dazu ausgelegt ist, durch die Intraokularlinse-Einsetzkassette (24) mittels einer Welle (16), welche den Ring (12) mit dem Griffstück (14) verbindet, eingesetzt zu werden.

## Revendications

1. Dispositif de capsulo-rhexis (10) adapté pour être inséré dans une incision oculaire, comprenant :
a) une poignée (14) à une première extrémité du dispositif ;
b) un anneau souple (12) ayant une surface postérieure (21) à l'autre extrémité du dispositif ;
c) un fil résistif en tant qu'élément chauffant (19), adapté pour être connecté à une source de courant électrique ;
**caractérisé en ce que,**
l'élément chauffant (19) est enrobé dans l'anneau au niveau de sa surface postérieure (21), de manière à faire saillie vers l'extérieur à une petite distance de la surface (21).

2. Dispositif selon la revendication 1, dans lequel l'anneau souple (12) est constitué d'un matériau relativement souple, comprenant un élastomère, un matériau acrylique ou un matériau thermoplastique.

3. Dispositif selon la revendication 2, dans lequel le matériau souple utilisé pour former l'anneau (12) a une mémoire de forme.

4. Dispositif selon la revendication 3, dans lequel l'anneau (12) peut être aplati de telle sorte qu'il peut être inséré dans un oeil à travers une incision inférieure à 2 mm.

5. Kit comprenant le dispositif selon l'une quelconque des revendications 1 à 4, et une cartouche d'insertion de lentille intraoculaire (24), dans lequel le dispositif est adapté pour être inséré à travers la cartouche d'insertion de lentille intraoculaire (24) au moyen d'un arbre (16) reliant l'anneau (12) à la poignée (14).
